# EUROPÄISCHE PATENTANMELDUNG

(11) **EP 1 491 155 A1**
(43) Veröffentlichungstag der Anmeldung: **29.12.2004**
(21) Anmeldenummer: 03405479.1
(22) Anmeldetag: 27.06.2003
(51) Int. Cl.: A61B 17/32

(54) **Schiebeschaftinstrument für chirurgische Zwecke, insbesondere Knochen- und Gewebestanze**

(71) Anmelder: Ulrich AG, 9015 St. Gallen (CH)
(72) Erfinder: Radlinger, Manfred, 9000 St Gallen (CH); Urlich, Christoph H., 9053 Teufen (CH)
(74) Vertreter: Wenger, René

(57) **Zusammenfassung**

Das Schiebeschaftinstrument (1) weist einen Schaft (2) auf, der an einem Ende (3) fest mit einem Schaftgriff (4) verbunden ist. Auf dem Schaft ist ein Schieber (5) in einer Längsführung (6, 6') verschiebbar gelagert. Der Schieber steht mit einem unter Federvorspannung stehenden Schiebergriff (8) in Wirkverbindung und kann durch Betätigen des Schiebergriffs aus einer Ruhestellung (A) in eine Arbeitsstellung (B) verschoben werden. Der Schieber ist mit Hilfe einer Begrenzungsschraube (9) in der Ruhestellung begrenzbar, welche gleichzeitig den Schieber in der Längsführung verriegelt. Die Begrenzungsschraube kann auf einfachste Weise durch Drehen zwischen einer Verriegelungsposition und einer Ausfahrposition verstellt werden, welche das Ausfahren des Schiebers aus der Längsführung und das Erreichen einer Reinigungsstellung ermöglicht.

## Beschreibung

Die Erfindung betrifft ein Schiebeschaftinstrument für chirurgische Zwecke, insbesondere Knochen- und Gewebestanze gemäss dem Oberbegriff von Anspruch 1. Derartige Instrumente dienen dazu, an teilweise schwer zugänglichen Körperstellen Resektionen vorzunehmen oder Gewebeproben zu entnehmen, wie beispielsweise bei der Laminektomie.

Gattungsmässig vergleichbare Instrumente sind in der Chirurgie seit längerer Zeit bekannt und gebräuchlich. Ein grundsätzliches Problem bei chirurgischen Instrumenten ist ersichtlicherweise die Reinigung und Sterilisation nach jedem Gebrauch. Einer optimalen mechanischen Funktion steht daher das Erfordernis gegenüber, dass das Instrument möglichst einfach zerlegt werden kann und dass keine schwer zugänglichen Stellen vorhanden sind. Bei einem Schiebeschaftinstrument muss in jedem Fall der Schieber vom Schaft entfernt werden können, da sonst eine Reinigung und Sterilisation nicht möglich ist.

Durch die DE 197 48 369 ist ein Schiebeschaftinstrument bekannt geworden, bei dem der Schieber in einer bestimmten Betriebsstellung ganz aus dem ihn betätigenden Gelenkhebel ausgefahren werden kann. Diese Betriebsstellung kann nur erreicht werden, wenn vorher eine Verriegelung am Schaftende gelöst wird. Der Nachteil bei diesem Instrument besteht darin, dass der Schieber nach der Entfernung vom Schaft nicht mehr mit dem restlichen Instrument verbunden ist, was die Zuordnung beim Sterilisieren erschwert.

Durch die DE 199 21 614 ist eine Knochenzange bekannt geworden, bei welcher der Schieber in der Reinigungsstellung über eine Koppel unverlierbar mit dem übrigen Instrument verbunden bleibt.

Ein Ausfahren aus der Längsführung und Aufschwenken des Schiebers ist auch hier nur möglich, wenn vorher ein Freigabe- bzw. Löseelement betätigt wird. Dieses besteht aus einem Lösedrehknopf der seitlich im Gelenkbereich der beiden Griffe angeordnet ist und der gegen den Widerstand einer Kugelarretierung in eine Lösestellung gedreht werden kann.

Beim chirurgischen Instrument gemäss EP 1 092 397 ist der Schieber ebenfalls in eine Reinigungsstellung aufschwenkbar, in welcher er unverlierbar gehalten ist. Die Verbindung zum restlichen Instrument wird jedoch nicht über eine separate Koppel, sondern direkt über den Schieberhebel aufrechterhalten. Die Sicherung des Schiebers in seiner Längsführung erfolgt ebenfalls über einen Riegel, der an verschiedenen Stellen am Instrument angeordnet sein kann und der den Schieberweg gegen die Kraft der Federvorspannung begrenzt.

Ein Nachteil aller bekannten Schiebeschaftinstrumente besteht darin, dass die Verriegelung zur Sicherung des Schiebers in seiner Längsführung ihrerseits in irgend einer Form gesichert werden muss, um ein unbeabsichtigtes Lösen zu vermeiden. Zu diesem Zweck werden Kugelarretierungen oder verschiedene Arten von Federsicherungen vorgeschlagen, die jedoch allesamt den Nachteil haben, dass sie schwer zugängliche Stellen bilden. Ausserdem handelt es sich bei den Riegeln um komplizierte Bauteile, welche die Herstellung und Montage verteuern. Es ist daher eine Aufgabe der Erfindung, ein Schiebeschaftinstrument der Eingangs genannten Art zu schaffen, bei dem eine Sicherung der längs verschiebbaren Verbindung zwischen Schieber und Schaft auf einfachste Weise gewährleistet ist, ohne die Funktionalität des Instruments zu beeinträchtigten. Die Verriegelung soll ausserdem bei einem hohen Grad von Sicherheit einfach zu betätigen sein.

Diese Aufgabe wird erfindungsgemäss mit einem Schiebeschaftinstrument gelöst, das die Merkmale im Anspruch 1 aufweist. Die in die Längsführung eingreifende Begrenzungsschraube als Begrenzungsmittel ist äusserst einfach zu betätigen und benötigt keinerlei zusätzliche Sicherungsmittel, um sie in der Verriegelungsposition zu halten. Das Einschraubdrehmoment beim Erreichen der Verriegelungsposition genügt, um diese Position auch beim Arbeiten mit dem Werkzeug zu halten. In der Ruhestellung wird die Begrenzungsschraube zusätzlich durch den unter Federvorspannung am Ende des Schraubenbolzens anliegenden Abschnitt des Schiebers gesichert. Die Ausfahrposition kann durch wenige Drehungen der Begrenzungsschraube erreicht werden, wozu lediglich eine geringfügige Bewegung des Schiebers aus der Ruhestellung gegen die Arbeitsstellung erforderlich ist, um das Lösen der Begrenzungsschraube zu erleichtern.

Die Begrenzungsschraube ist vorteilhaft eine Rändelschraube, die in eine quer zur Bewegungsrichtung des Schiebers verlaufende Gewindebohrung auf der Unterseite des Schaftes eingeschraubt ist. Dabei ist es zweckmässig, wenn der Kopf der Rändelschraube einen Durchmesser aufweist, der grösser ist als die Breite des Schaftes bzw. des Schiebers. Auf diese Weise lässt sich die Begrenzungsschraube gut anfassen und betätigen, ohne dass sie beim Arbeiten des Chirurgen störend in Erscheinung tritt. Selbstverständlich wäre aber auch eine seitliche Anordnung am Schaft oder am Schieber oder auch eine Anordnung auf der Oberseite des Schiebers denkbar. Anstelle einer Rändelschraube könnte es sich auch um eine Flügelschraube oder um eine andere manuell leicht zu betätigende Schraube handeln.

Weitere Vorteile können erreicht werden, wenn die Begrenzungsschraube in eine in den Schaft oder in den Schieber eingesetzte Gewindebuchse eingeschraubt ist. Die Gewindebuchse hat fabrikationstechnische Vorteile, weil dadurch am Schaft bzw. am Schieber lediglich eine einfache Bohrung angebracht werden muss. Der Aufwendige Vorgang des Gewindeschneidens lässt sich an der Gewindebuchse wesentlich einfacher vornehmen. Die Gewindebuchse kann mit einem leichten Presssitz in die Bohrung eingepresst und mit einer Laserschweissung fixiert werden.

Die Begrenzungsschraube kann ausserdem durch ein Sicherungselement unverlierbar in ihrer Gewindebohrung gehalten sein. Dabei kann es sich um einen Sicherungskragen handeln, der auf das Ende des Schraubenbolzens aufgeschweisst wird. Auf diese Weise besteht nicht die Gefahr, dass die Sicherungsschraube beispielsweise während des Sterilisiervorgangs verloren geht.

Damit der Schraubenhub zwischen der Verriegelungsposition und der Ausfahrposition möglichst rasch zurückgelegt werden kann, weist die Begrenzungsschraube vorzugsweise ein wenigstens zweigängiges Gewinde auf.

Die Längsführung zwischen Schaft und Schieber weist vorteilhaft wenigstens zwei Führungsabschnitte mit je einer T-förmigen Führungsnut im Schaft und mit je einem T-förmigen Führungselement am Schieber auf. Selbstverständlich wäre es aber auch denkbar, dass die T-förmige Führungsnut am Schieber und das T-förmige Führungselement am Schaft angeordnet ist. Anstelle des T-förmigen Querschnitts könnte die Längsführung aber beispielsweise auch als Schwalbenschwanzführung oder anderweitig ausgebildet sein.

Weitere Einzelmerkmale und Vorteile der Erfindung ergeben sich aus der nachfolgenden Beschreibung eines Ausführungsbeispiels und aus den Zeichnungen. Es zeigen:
- Figur 1: eine Seitenansicht eines Schiebeschaftinstruments in der Ruhestellung bzw. in der Arbeitsstellung,
- Figur 2: das Instrument gemäss Figur 1 in der Reinigungsstellung,
- Figur 3: einen Querschnitt durch das Detail X gemäss Figur 1,
- Figur 4: eine Draufsicht auf die Schaftpartie gemäss Figur 3, und
- Figur 5: die Detaildarstellung gemäss Figur 3 mit ausgefahrenem Schieber.

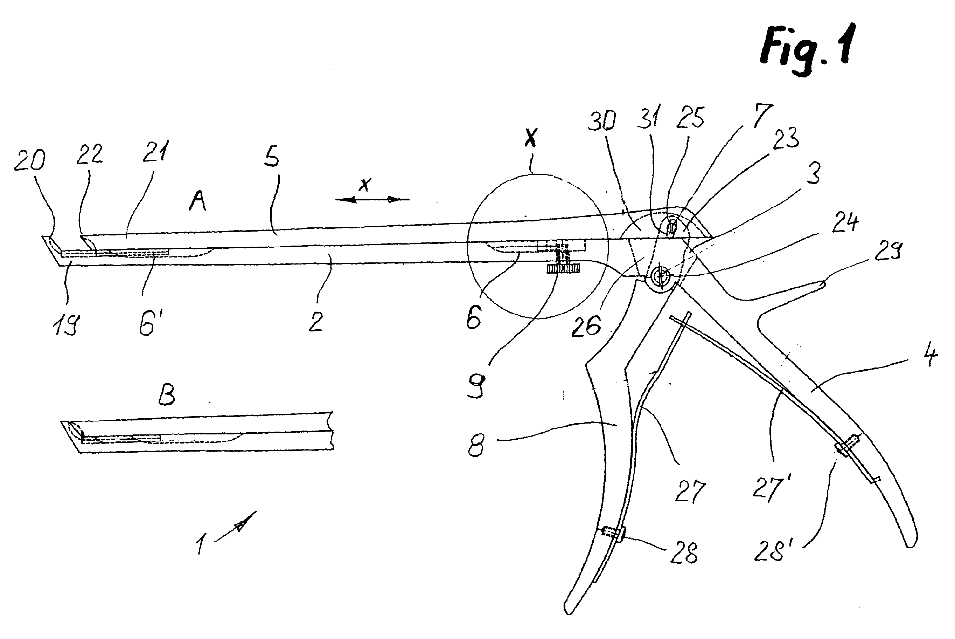

Wie in Figur 1 dargestellt, besteht ein allgemein mit 1 bezeichnetes Schiebeschaftinstrument im wesentlichen aus einem Schaft 2 und aus einem in Pfeilrichtung x in Längsführungen 6, 6' auf dem Schaft verschiebbaren Schieber 5. Dabei kann der Schieber aus einer Ruhestellung A in eine Arbeitsstellung B verschoben werden. Im vorliegenden Fall handelt es sich beim Instrument um eine Laminektomiestanze, bei welcher am werkzeugseitigen Ende 19 des Schaftes ein Stanzwiderlager 20 und am werkzeugseitigen Ende 21 des Schieber ein Stanzabschnitt 22 angeordnet ist. Art und Konfiguration des Werkzeuges können jedoch je nach Anwendungszweck variieren und es wäre beispielsweise auch denkbar, dass über den Schieber ein scherenartiges Schneidewerkzeug betätigt wird.

Der Schaft 2 ist an seinem griffseitigen Ende 3 einstückig mit einem Schaftgriff 4 verbunden. Im Bereich des Übergangs zwischen Schaft und Schaftgriff ist ein durchgehender Schlitz 26 angeordnet. In diesem Bereich ist ausserdem ein Schiebergriff 8 an einem Drehgelenk 3 schwenkbar gelagert.

Der Schiebergriff 8 ist mit einem den Schlitz 26 durchdringenden Schieberhebel 23 versehen, der an seinem Ende ein Langloch 31 aufweist. Der Schieber 5 verfügt an seinem griffseitigen Ende 7 über eine mit dem Schlitz 26 korrespondierende Ausnehmung 30 und über einen in das Langloch 31 eingreifenden Mitnehmerbolzen 25. Die Schwenkbewegung des Schieberhebels 23 um das Drehgelenk 24 wird somit ersichtlicherweise in eine Längsbewegung am Schaft in Pfeilrichtung x umgewandelt.

Schaftgriff 4 und Schiebergriff 8 werden durch die beiden ineinander verschränkten Blattfedern 27, 27' auseinander gespreizt, welche mittels Schrauben 28 bzw. 28' an den beiden Griffen fixiert sind. Infolge der Hebelmechanik ist somit der Schieber 5 stets in die Ruhestellung A vorgespannt. Der Schaftgriff 4 verfügt ausserdem noch über einen Griffdorn 29, der einen sicheren Griff gewährleistet und ein Abgleiten der Hand verhindert.

Die Begrenzung des Schiebers 5 in der Ruhestellung A erfolgt mit Hilfe der Begrenzungsschraube 9, die auf der Unterseite des Schaftes 2 eingeschraubt ist und deren Ende in die Längsführung 6 eingreift. Die Begrenzungsschraube verriegelt gleichzeitig den Schieber 5 in der Längsführung, wie nachstehenden noch genauer beschrieben wird.

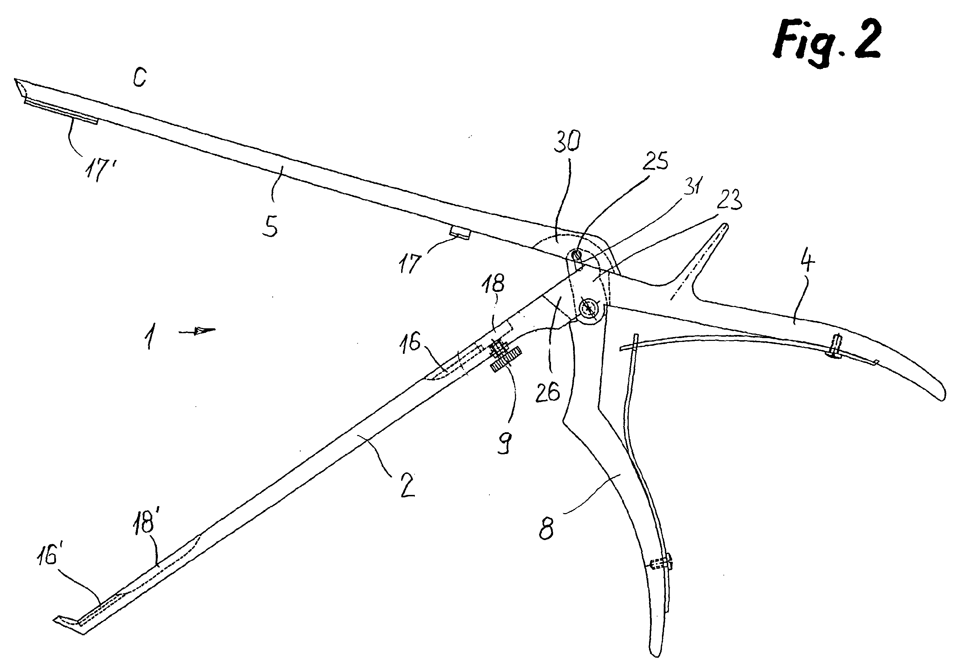

Figur 2 zeigt die Reinigungsstellung C des Schiebeschaftinstruments 1, die nach dem Verstellen der Begrenzungsschraube 9 in die Ausfahrposition erreicht werden kann. Dabei wird der Schiebergriff 8 unter der Federvorspannung noch etwas weiter aufgespreizt, bis ein Ausfahren der Führungselemente 17, 17' aus den Führungsnuten 16, 16' möglich ist. Der Schieber 5 kann danach um eine bestimmten Schwenkwinkel gedreht werden, bleibt jedoch fest mit dem Schieberhebel 23 verbunden. In dieser Stellung kann das Instrument in einen Sterilisierkorb zu anderen Instrumenten gelegt werden, wobei es stets zu einer Einheit verbunden bleibt.

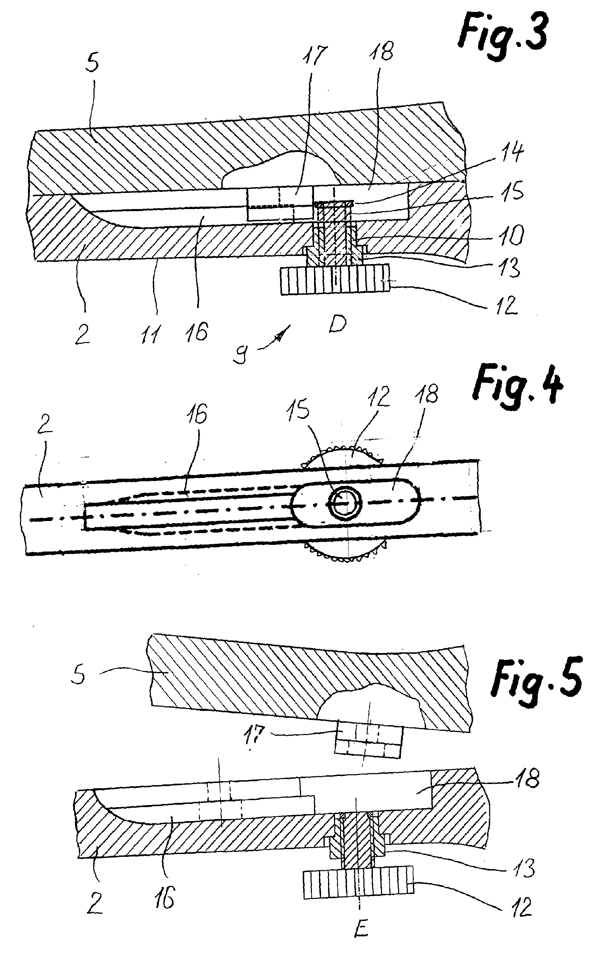

Anhand der Figuren 3 bis 5 werden weitere Einzelheiten der Begrenzungsschraube 9 beschrieben. Bei der Begrenzungsschraube handelt es sich um eine Rändelschraube, deren Schraubenkopf 12 einen Durchmesser aufweist, der grösser ist als die Breite des Schaftes bzw. des Schiebers. Wie insbesondere aus Figur 4 ersichtlich ist, kann der Schraubenkopf 12 auf diese Weise an den seitlich vorstehenden Segmenten gut angefasst werden.

Der Schraubenbolzen 15 ist an seinem freien Ende fest mit einem umlaufenden Sicherungsring verschweisst, der ein Verlieren der Begrenzungsschraube verhindert. Die Gewindebohrung 10 für den Schraubenbolzen 15 ist in einer Gewindebuchse 13 angeordnet. Diese ist auf der Unterseite 11 des Schaftes in eine Bohrung eingepresst bzw. eingeschweisst. Der Gewindebolzen kann beispielsweise ein zweigängiges metrisches Gewinde mit 3 mm Durchmesser aufweisen.

Bei der in Figur 3 dargestellten Verriegelungsposition D greift das Ende des Schraubenbolzens 15 in die Längsführung 6, welche insgesamt aus einer T-förmigen Führungsnut 16, einem T-förmigen Führungselement 17 und einem Einführabschnitt 18 besteht. Die Breite des Einführabschnitts 18 ist so dimensioniert, dass das Führungselement 17 zwar in Längsrichtung, aber nicht quer dazu geführt ist.

Aus Figur 3 ist ersichtlich, dass das Führungselement 17 etwa mit der Hälfte seiner Länge in die Führungsnut 16 eingreift, jedoch durch das Ende des Schraubenbolzens 15 am Zurückgleiten in den Einführabschnitt gehindert wird. In dieser Position ist ein Abheben des Schiebers 5 vom Schaft 2 ersichtlicherweise nicht möglich.

Nach dem Verstellen der Begrenzungsschraube 9 in die in Figur 5 dargestellte Ausfahrposition E kann das Führungselement 17 über seine gesamte Länge in den Einführabschnitt 18 zurückgleiten, wodurch ein Aufschwenken des Schiebers 5 ermöglicht wird. In Figur 5 sind auch noch die T-förmigen Querschnitte der Führungsnut 16 bzw. des Führungselements 17 angedeutet. Nach dem Reinigen und Sterilisieren des Instruments wird auf umgekehrte Weise verfahren. Der Schieber 5 wird auf den Schaft 2 zurückgeklappt, wobei das Führungselement 17 in den Einführabschnitt 18 eintaucht. Durch Betätigen des Schiebergriffs 8 (Figur 1) wird der Schieber 5 wenigstens so weit gegen die Arbeitsstellung B in Pfeilrichtung x verschoben, bis das Führungselement 17 in die Führungsnut 16 eingreift und ein Verstellen der Begrenzungsschraube 9 in die Verriegelungsposition D möglich ist.

## Patentansprüche

1. Schiebeschaftinstrument für chirurgische Zwecke, insbesondere Knochen- und Gewebestanze, mit einem Schaft (2), der an einem Ende (3) fest mit einem Schaftgriff (4) verbunden ist und mit einem Schieber (5), der auf dem Schaft in einer Längsführung (6, 6') verschiebbar gelagert ist und der an einem Ende (7) derart mit einem unter Federvorspannung stehenden Schiebergriff (8) in Wirkverbindung steht, dass durch Betätigen des Schiebergriffs der Schieber gegen die Federvorspannung aus einer Ruhestellung (A) in eine Arbeitsstellung (B) verschiebbar ist, wobei die Ruhestellung durch ein Begrenzungsmittel begrenzbar ist, das den Schieber (5) gleichzeitig in der Längsführung verriegelt und wobei durch Lösen des Begrenzungsmittels der Schieber aus der Längsführung ausfahrbar und vorzugsweise unverlierbar gehalten in eine vom Schaft (2) abgehobene Reinigungsstellung (C) schwenkbar ist, **dadurch gekennzeichnet, dass** das Begrenzungsmittel eine am Schaft (2) oder am Schieber angeordnete Begrenzungsschraube (9) ist, welche in die Längsführung (6) eingreift und durch Drehen zwischen einer Verriegelungsposition (D) und einer Ausfahrposition (E) verstellbar ist.

2. Schiebeschaftinstrument nach Anspruch 1, **dadurch gekennzeichnet, dass** die Begrenzungsschraube (9) eine Rändelschraube ist, die in eine quer zur Bewegungsrichtung des Schiebers (5) verlaufende Gewindebohrung (10) auf der Unterseite (11) des Schafts eingeschraubt ist.

3. Schiebeschaftinstrument nach Anspruch 2, **dadurch gekennzeichnet, dass** der Kopf (12) der Rändelschraube einen Durchmesser ausweist, der grösser ist als die Breite des Schaftes bzw. des Schiebers.

4. Schiebeschaftinstrument nach einem der Ansprüche 1 bis 3, **dadurch gekennzeichnet, dass** die Begrenzungsschraube (9) in eine in den Schaft eingesetzte Gewindebuchse (13) eingeschraubt ist.

5. Schiebeschaftinstrument nach einem der Ansprüche 1 bis 4, **dadurch gekennzeichnet, dass** die Begrenzungsschraube (9) durch ein Sicherungselement (14) unverlierbar in ihrer Gewindebohrung (10) gehalten ist.

6. Schiebeschaftinstrument nach einem der Ansprüche 1 bis 5, **dadurch gekennzeichnet, dass** die Begrenzungsschraube (9) ein wenigstens zweigängiges Gewinde aufweist.

7. Schiebeschaftinstrument nach einem der Ansprüche 1 bis 6, **dadurch gekennzeichnet, dass** die Längsführung (6, 6') wenigstens zwei Führungsabschnitte mit je einer T-förmigen Führungsnut (16, 16') im Schaft (2) und je einem T-förmigen Führungselement (17, 17') am Schieber (5) aufweist.
